# FASCICULE DE BREVET EUROPEEN

(11) **EP 2 454 991 B1**
(45) Date de publication et mention de la délivrance du brevet: **19.03.2014**
(21) Numéro de dépôt: 11185904.7
(22) Date de dépôt: 20.10.2011
(51) Int. Cl.: A61B 5/053

(54) **Appareil de bio-impedancemetrie ergonomique**
Ergonomisches Gerät zur Messung der Bio-Impedanz
Ergonomic bio-impedance measurement device

(30) Priorité: 18.11.2010 FR 1059489
(43) Date de publication de la demande: 23.05.2012
(73) Titulaire: Aminogram, 13600 La Ciotat (FR)
(72) Inventeur: Letourneur, Alain, 83000 TOULON (FR)
(74) Mandataire: Domange, Maxime

(56) Documents cités:
- JP-A- 10 127 593
- JP-A- 11 019 059
- JP-A- 11 128 198
- JP-A- 2001 104 270
- US-A1- 2004 059 242

## Description

### Arrière-plan de l'invention

La présente invention se rapporte au domaine général des appareils de bio-impédancemétrie.

De tels appareils, par exemple l'appareil commercialisé par la société déposante, appelé AMINOSTATS BIO-ZM II, met en oeuvre une mesure de bio-impédancemétrie. Il s'agit d'une technique indolore, rapide et économique, permettant de connaître en quelques minutes la composition corporelle d'un sujet, sous la forme d'une évaluation de la quantité d'eau globale, d'une évaluation de la masse grasse et de la masse maigre.

Le principe de la mesure consiste à faire passer un courant électrique au travers du corps d'un sujet et de mesurer l'impédance observée pour une fréquence de courant donnée. Le courant auquel est soumis le sujet est de faible valeur, typiquement entre 0,2 à 1 mA, et ne sera, par conséquent, pas ressenti par celle-ci. La mesure de l'impédance à une fréquence donnée permet d'évaluer la quantité de fluide que le courant a pu traverser. Les fluides, sang, lymphe ou intracellulaires, urine, etc., se comportent comme des électrolytes. Les graisses se comportent sensiblement comme des isolants. La mesure de l'impédance à une fréquence donnée va permettre d'évaluer la quantité de fluide que le courant a pu traverser.

De tels appareils sont avant tout des produits professionnels mais la tendance est de proposer des appareils grand public permettant de faire de telles mesures.

Au sein des appareils professionnels, il existe deux types de mesures : en mono-fréquence et en multifréquence.

L'utilisation d'une seule fréquence, généralement égale ou supérieure à 50 kHz, pour le courant émis dans le corps d'un patient permet d'accéder uniquement à l'eau globale, à la masse grasse et à la masse maigre. La masse maigre est constituée de l'eau et des tissus non graisseux. Seules les fréquences inférieures ou égales à 5 kHz permettent de dissocier l'eau intra et extracellulaire de l'eau globale.

Les appareils grand public connus n'utilisent que la mono-fréquence, du fait qu'une telle mesure est très simple à réaliser et n'exige pas une grande précision de positionnement des électrodes servant à la mesure.

Il existe aujourd'hui des pèse-personnes en grande surface qui donnent le poids global et la masse graisseuse, tels que décrit dans JP 10-127593 et JP 11-128198. Cette mesure de masse graisseuse est effectuée par bio-impédancemétrie de type mono-fréquence et, uniquement, sur le bas du corps. Bien que ces mesures de masse graisseuse permettent de dégager une tendance, ces outils sont largement insuffisants à cause de leur manque de précision et de fiabilité.

On connait aussi des appareils de mesure de graisse, effectuant la mesure au niveau des mains, tels que décrit dans JP 11-019059 et JP 2001-104270.

On connaît aussi des appareils du type pèse-personne sur lesquels le sujet vient placer les deux pieds et vient saisir deux poignées avec les mains. La mesure se fait alors du bas du corps au haut du corps mais reste approximative car le positionnement des contacts permet une transmission du courant plus approximative et non optimale.

Quelques appareils de ce type, réservés aux professionnels de part leur prix très élevé, utilisent les mesures multifréquence. Cependant ils ne semblent pas être d'une aussi bonne précision que les appareils de type filaire.

Les seuls appareils aujourd'hui connus, qui réalisent des mesures multifréquence d'une très grande précision, sont des appareils professionnels dits de type filaire qui comportent des câbles au bout desquels sont placées les électrodes spécifiques pour les mains et les pieds, tels que décrits dans US 2004/0059242. Ces appareils connus exigent donc que les électrodes soient positionnées par un professionnel. Aucun appareil connu destiné au grand public n'utilise les mesures multifréquence en raison d'un coût trop élevé et la difficulté à respecter le bon positionnement des électrodes.

Les mesures multifréquence exploitent le fait que les tissus nobles, typiquement des protéines, ont une réaction biodynamique de défense au passage du courant spécifique. Cette réaction est plus ou moins efficace en fonction de la fréquence du courant et du tissu lui-même.

Ainsi, la membrane des cellules sont très résistives et se comportent donc comme autant de diélectriques séparant de petits conducteurs constitués par les fluides internes des cellules. Un comportement réactif des tissus nobles plus ou moins important sera donc observé en fonction de la fréquence du courant utilisée pour la mesure d'impédance.

L'utilisation d'une pluralité de fréquences de courant permet d'accéder à une grande précision de mesure et à de nouvelles informations qui peuvent s'avérer cruciales dans l'établissement des recommandations aux patients.

En l'occurrence, l'utilisation d'une pluralité de fréquences permet d'accéder à la masse osseuse, à la masse sèche hors graisse, à la quantité d'eau intracellulaire et à la quantité d'eau extracellulaire, au taux d'hydratation hors graisse et, encore, à d'autres paramètres utiles pour établir le profil d'un sujet.

En effet, des équations basées sur des études statistiques et des comparatifs avec d'autres procédés d'évaluation permettent de déduire des mesures des quantités d'eau totale, d'eau extracellulaire et intracellulaire, de masse grasse et de masse maigre. Certaines équations permettent même une évaluation du squelette.

Les résultats obtenus dépendent du patient lui-même, du soin pris pour prendre la mesure, du matériel de mesure et des équations utilisées. Pour des individus moyens, en bonne santé, on peut obtenir des résultats précis et fiables permettant de les considérer comme des valeurs vraies.

Dans tous les autres cas, les résultats seront indicatifs mais permettront des constats d'évolution. Pour des mesures fiables, il faut garder tous les éléments constants, en particulier utiliser toujours le même appareil, positionner les électrodes avec précision, s'assurer que le patient soit dans la même position et éviter les changements importants d'horaires de la mesure.

Le frein principal d'utilisation de mesures multifréquence dans les appareils grand public est la nécessité d'un positionnement précis et correct des électrodes sur le corps du sujet. Il n'existe pas à ce jour d'appareil permettant un tel positionnement reproductible et correct des électrodes sur le corps du sujet par une personne non qualifiée.

### Objet et résumé de l'invention

La présente invention a donc pour but principal de pallier de tels inconvénients, en proposant un appareil de prise de mesure de bio-impédancemétrie tel que revendiqué à la revendication 1.

Un tel appareil présente un unique support destiné à accueillir une main dans un positionnement prédéfini pour supporter l'ensemble des quatre électrodes. En outre, le support est tel qu'une fois le support ayant accueilli la main et l'élément de maintien ayant été utilisé pour maintenir la main dans la position prédéfinie, les deux électrodes hautes sont positionnées directement en deux points distincts de la main. Dans la mesure où les électrodes sont directement supportées par le support et que la main vient prendre une position prédéfinie par rapport au support, la position des électrodes par rapport à la main est reproductible chaque fois que la même main vient se positionner dans le support dans sa position prédéfinie.

Le support muni de ces extensions supporte aussi les deux électrodes basses permettant de faire les contacts au niveau du bas du corps de l'utilisateur. Le positionnement reproductible des électrodes basses est assuré par la structure en pince générée par la présence des extensions. Une telle structure permet d'assurer que, pour une même main positionnée dans le support, les électrodes basses soient dans des positions relatives identiques et reproductibles.

Ainsi, l'utilisateur a seulement à positionner la structure en pince par rapport à la cheville ou au pied en amenant sa main vers son pied ou sa cheville en s'assurant que la structure en pince vienne bien pincer sa cheville ou son pied en un endroit particulier qui lui est enseigné auparavant. Les positions relatives des électrodes basses placées sur le support étant toujours identiques, on diminue beaucoup les risques de positionnement incorrect.

Ainsi, l'invention permet un positionnement prédéfini par rapport à la main très précis pour les électrodes hautes, et un positionnement très reproductible au niveau du pied, dès lors que l'utilisateur a reçu un enseignement pour savoir positionner la structure en pince par rapport à sa cheville ou son pied.

Une telle réalisation d'un appareil de prise de mesures de bio-impédancemétrie est tout à fait adaptée à des prises de mesures multi-fréquentielles. En effet, le positionnement précis, correct et reproductible des électrodes hautes et basses est assuré grâce à l'invention.

Selon une réalisation préférentielle, le support est tel qu'une des électrodes hautes est destinée à être positionnée au niveau du poignet et l'autre électrode haute est destinée à être positionnée au niveau d'un des doigts de la main, les deux électrodes étant séparées d'une distance supérieure ou égale à 11 centimètres sur le support.

Cette réalisation permet de contrer tout phénomène d'interférence entre les deux électrodes hautes et d'avoir une distance entre les deux électrodes hautes minimale pour assurer une mesure correcte.

Avantageusement, le module est installé dans un boitier positionné au niveau du poignet.

Cette caractéristique permet de positionner la partie de l'appareil a priori la plus volumineuse au niveau du poignet. On assure ainsi une installation facile de l'appareil selon l'invention, puisqu'il est facile de poser le boitier au niveau du poignet, avant de refermer des éléments de maintien avec la main opposée.

Selon une réalisation préférentielle, l'élément de maintien est un bracelet élastique ou non, destiné à enserrer le poignet.

Une telle réalisation permet un maintien robuste de l'appareil sur la main de l'utilisateur et permet, également, une mise en oeuvre du maintien facile et naturelle. En effet, les utilisateurs de tels systèmes grand public de prise de mesures biologiques sont habitués à positionner un appareil sur le poignet, typiquement un tensiomètre, un cardiofréquencemètre, etc.

Avantageusement, le bracelet supporte une des électrodes hautes.

Cette caractéristique permet d'utiliser la fermeture du bracelet pour appuyer l'électrode haute sur un point du dos de la main.

Dans une réalisation avantageuse, une des deux extensions porte également une des électrodes hautes, cette extension étant placée sur le support de telle manière qu'une fois la main positionnée dans le support, un des doigts est naturellement positionné au niveau de l'électrode.

Cette réalisation permet d'utiliser une extension pour porter deux électrodes, une basse et une haute. En outre, cette réalisation permet qu'une des extensions de la structure formant pince, soit en contact avec un des doigts de l'utilisateur, ce qui aide beaucoup à l'enseignement et à la mémorisation du positionnement de la main et, donc, du support par rapport à la cheville ou au pied.

Dans une première réalisation, les deux extensions constituent une pince flexible adaptée pour pincer la cheville ou le pied et mettre ainsi en contact les électrodes portés par les extensions en contact avec la peau de part et d'autre de la cheville ou du pied.

Cette réalisation exige que la pince flexible soit formée directement sur le support et présente la fonction, elle-même, de pincer la cheville ou le pied.

Selon un second principe de réalisation préférentiel, que les deux extensions sont dactylo-profilées pour recevoir deux doigts de la main.

Avec ce principe de réalisation, ce sont deux doigts de la main qui constituent la pince qui va venir se positionner par rapport au pied ou à la cheville. Cette réalisation permet que, après un enseignement particulier prodigué à l'utilisateur, celui-ci soit totalement autonome pour positionner ses deux doigts, par exemple le pouce et un autre doigt de la main, index ou majeur, par rapport typiquement à la malléole externe et à la malléole interne de sa propre cheville. Cette réalisation présente l'avantage de ne pas nécessiter la présence de deux extensions rigides qui nuisent à la compacité de l'appareil selon l'invention.

Dans une réalisation, le support a la forme d'un gant à au moins deux doigts, par exemple le pouce et un autre des doigts de la main, un des doigts du gant portant une électrode haute sur sa face interne et une électrode basse sur sa face externe, l'autre doigt portant une électrode basse sur sa face externe.

Avec cette réalisation, le support est un gant, support naturel pour une main. Le gant peut être réalisé à partir de matériaux plus ou moins extensibles pour s'adapter à des mains de tailles diverses. Plusieurs tailles de gants pourront aussi être disponibles. L'utilisation d'un des doigts du gant pour porter à la fois une électrode haute et une électrode basse, permet d'assurer une grande simplicité à l'appareil selon l'invention.

On note ici, cependant, que le gant pourra tout à fait comprendre trois doigts pour positionner, sur trois doigts distincts, l'électrode haute et les deux électrodes basses. Cela peut, d'ailleurs, être nécessaire dans le cas où les fréquences utilisées engendrent des problèmes d'interférences entre l'électrode haute positionnée sur la face interne du gant et l'électrode basse positionnée sur la face externe du gant, au niveau du même doigt que celui où est positionnée l'électrode haute.

Dans un mode de réalisation préférentiel, le support est réalisé à partir d'une plaque de matériau souple où est découpée une forme spécifique formant un bracelet de maintien au niveau duquel sont supportés le module et une première électrode haute, une paume et deux extensions à l'extrémité desquelles sont placés des capuchons pour recevoir deux doigts de la main, un premier capuchon étant muni d'une électrode basse sur sa face externe, l'autre capuchon étant muni d'une électrode basse sur sa face externe et d'une électrode haute placée sur la face interne du capuchon.

Cette réalisation, particulièrement simple, assure une très bonne compacité de l'appareil selon l'invention ainsi qu'un maintien très fidèle et reproductible de la main et donc des doigts par rapport au support. Cette réalisation permet aussi d'utiliser un matériau élastique plan, ce qui simplifie beaucoup sa fabrication par rapport à l'utilisation d'un gant dans lequel on doit venir positionner une électrode sur la face interne. Cette réalisation permet aussi d'éviter la nécessité de devoir constituer deux extensions formant une pince flexible puisque la main est utilisée. L'utilisation de capuchons en extrémité des extensions permet une installation facile des électrodes mais aussi un remplacement et une maintenance facilités.

Avantageusement, la paume du support comprend au moins un orifice de placement par rapport au support d'un des autres doigts de la main autre que ceux devant être introduits dans les capuchons.

Un tel orifice de placement permet de positionner au moins un des autres doigts par rapport à la plaque de matériau souple, ce qui assure encore plus surement un positionnement correct de la main par rapport au support.

Dans une réalisation préférentielle, le module réalise des mesures de bio-impédancemétrie multi-fréquentielle.

Cette réalisation, qui a justifié la création de l'invention, est bien sûr une application préférentielle pour l'utilisation de l'invention.

Dans une réalisation particulière, le dispositif de traitement de données et/ou d'affichage est supporté par le support.

Cette réalisation consiste à intégrer totalement l'ensemble des éléments permettant la lecture des mesures de bio-impédancemétrie sur l'appareil selon l'invention lui-même.

Dans une réalisation particulière, les moyens d'émission comprennent des moyens de communication sans fil tels que l'appareil est relié par ces moyens de communication sans fil à un dispositif de traitement de données et/ou d'affichage.

Cette réalisation permet que l'appareil selon l'invention émette directement les données mesurées vers un dispositif de traitement de données et/ou d'affichage.

Dans une application avantageuse, le dispositif de traitement de données et/ou d'affichage est un serveur Internet.

Cette réalisation permet de centraliser les informations de prises de mesures de bio-impédancemétrie et de proposer des services particuliers utilisant ces prises de mesures.

Pour cela, le serveur Internet est apte à centraliser les mesures de bio-impédancemétrie par utilisateur et à calculer les indices corporels.

Cette caractéristique avantageuse permet de personnaliser les données retournées aux utilisateurs des appareils selon l'invention.

Ainsi, avantageusement, le serveur Internet est apte à déterminer un programme nutritionnel ou plan diététique à partir des indices corporels et à communiquer ce programme ou ce plan sur un ordinateur ou tout autre support numérique de l'utilisateur de l'appareil.

Cette réalisation permet de fournir, à partir d'une base de données mesurées sur le corps de l'utilisateur lui-même, des conseils personnalisés et adaptés au profil physique et physiologique de l'utilisateur.

### Brève description des dessins

D'autres caractéristiques et avantages de la présente invention ressortiront mieux à la lecture de la description qui va suivre, faite de manière illustrative et non limitative, en référence aux dessins annexés sur lesquels :
- la figure 1 montre un premier mode de réalisation basique de l'invention ;
- la figure 2 montre un second mode de réalisation selon l'invention ;
- les figures 3A et 3B montrent le principe des réalisations préférentielles de l'invention ;
- la figure 4 montre un troisième mode de réalisation selon l'invention ;
- les figures 5A et 5B montrent un quatrième mode de réalisation de l'invention ;
- les figures 6A et 6B montrent un cinquième mode de réalisation de l'invention ;
- les figures 7A et 7B montrent un sixième mode de réalisation de l'invention ;
- la figure 8 montre un schéma électrique simplifié représentant le principe de la mesure de bio-impédancemétrie quadripolaire.

### Description détaillée d'un mode de réalisation

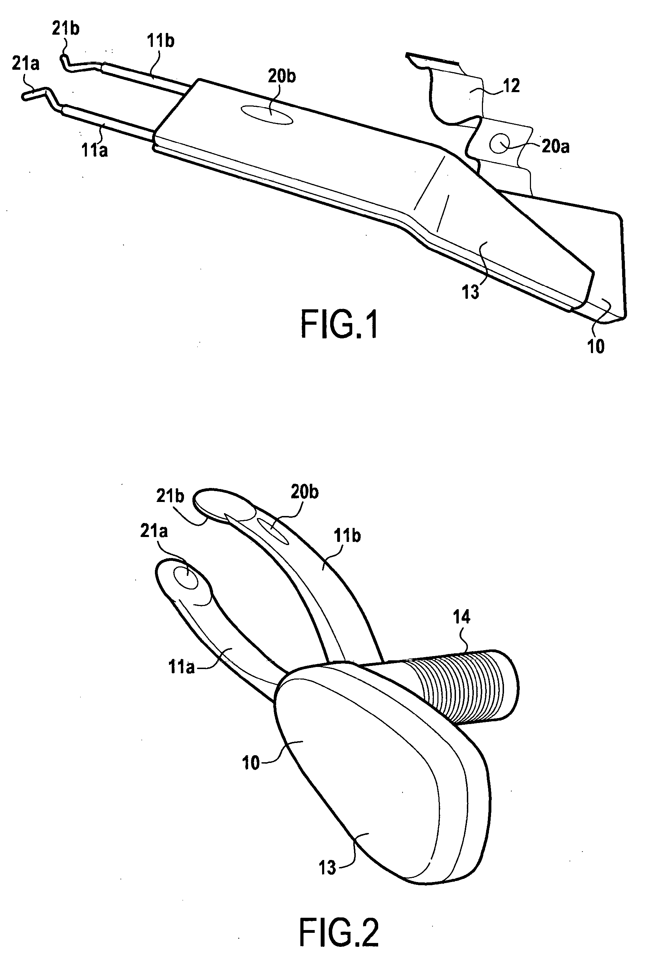

La figure 1 représente un premier mode de réalisation d'un appareil de prise de mesures de bio-impédancemétrie selon l'invention. L'appareil comprend un support 10 présentant deux extensions 11a et 11b. Dans cette réalisation, le support 10 inclut un boitier 13 contenant un module électronique apte à commander quatre électrodes pour réaliser une mesure de bio-impédancemétrie. Un élément de maintien 12 permet d'immobiliser la main par rapport au support 10.

L'appareil représenté sur la figure 1 est tel que l'utilisateur vient positionner sa main sur la face supérieure du support 10 et vient fermer l'élément de maintien 12 qui est un bracelet venant enserrer le poignet. Une fois le bracelet serré et fermé, la main est positionnée par rapport au support 10, de telle manière qu'elle peut attraper le support 10.

Dans le mode de réalisation de la figure 1, le bracelet de maintien 12 du support 10 supporte une première électrode haute 20a, alors que le support 10 lui-même supporte une deuxième électrode haute 20b, ici placé sur le boitier 13.

On comprend bien ici que, une fois la main positionnée sur le support 10 et l'élément de maintien 12 serré et fermé sur le poignet, un point du dos du poignet, proche du dos de la main, est en contact avec l'électrode 20a. Par ailleurs, on comprend aussi qu'un des doigts de l'utilisateur est naturellement positionné sur l'électrode 20b portée sur la face supérieure du support 10. Avantageusement, ce sera l'index qui sera placé sur l'électrode 20b.

Les deux extensions 11a et 11b supportent, quant à elles, deux électrodes dites basses 21a et 21b. On voit ici que les extensions 11a et 11b forment une structure en pince. Cette pince est adaptée pour pincer la cheville ou le pied de l'utilisateur, dès lors que le support 10 est amené vers la cheville ou le pied par la main de l'utilisateur. Les deux extensions 11a et 11b viennent alors pincer la cheville ou le pied en deux points distincts situés de part et d'autre de la cheville ou du pied.

Avantageusement, l'utilisateur aura été éduqué de manière à ce que les deux extensions 11a et 11b soient positionnées en des points particuliers, préférentiellement au niveau de la cheville où il est particulièrement avantageux de positionner les deux électrodes 21a et 21b au niveau de la malléole externe et de la malléole interne.

Le boîtier 13 comprend des moyens d'émission des mesures de bio-impédancemétrie vers un dispositif de traitement de données et/ou d'affichage. On constate ici qu'un dispositif de traitement de données pourrait être implémenté directement au sein du support 10, un dispositif d'affichage pouvant également être placé sur le support 10, par exemple au niveau de sa face intérieure qui se trouvera ainsi à l'intérieur de la main positionnée sur le support.

Le boîtier 13 sera avantageusement moulé en deux coquilles, intégrant l'ensemble de l'électronique.

La figure 2 montre un second mode de réalisation de l'invention où le support 10 est principalement constitué d'un boîtier 13 moulé en deux coquilles intégrant l'ensemble de l'électronique et d'une poignée 14.

Ici, l'utilisateur vient serrer la poignée 14 dans sa main. L'index vient alors naturellement se positionner au niveau d'une des deux extensions, ici l'extension 11b, munie d'une des électrodes hautes 20b. Les deux extensions 11a et 11b sont, par ailleurs, munies des deux électrodes basses 21a et 21b. Les deux extensions 11a et 11b forment une pince flexible que l'utilisateur va venir positionner par rapport à sa cheville ou son pied. L'électrode haute destinée au contact sur le dos de la main est placée avantageusement sur un bracelet du type de celui utilisé sur la figure 1 et fixé au boîtier 13 ou à la poignée 14. Un lien « scratché » sera avantageusement utilisé pour fermer le bracelet.

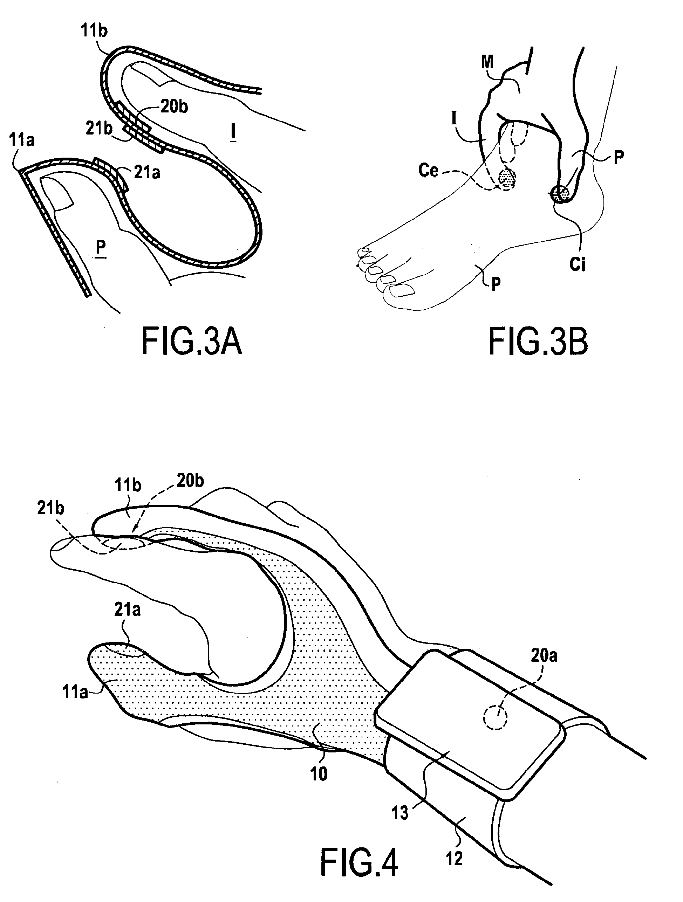

Les figures 3A et 3B montrent le principe d'un mode de réalisation préférentiel selon l'invention.

Comme montré sur la figure 3A, un premier doigt, ici le pouce P, porte une électrode basse 21a sur la face externe d'une extension 11a dans laquelle il est venu s'introduire. L'index I, placé dans une extension 11b, porte quant à lui, sur la face externe de l'extension 11b, la seconde électrode basse 21b, alors que la seconde électrode haute 20b est placée sur la face interne de l'extension 11b.

Ainsi la fonction mécanique du support est très simplifiée par l'utilisation de la main M elle-même, et, plus précisément, de deux doigts de la main M, pour former la structure en pince. Les réalisations présentées sur les figures suivantes partent ainsi du constat que le pouce et l'index ou le majeur forment une pince naturelle. On remarque aussi que la paire majeur/index peut aussi être utilisée puisque ces deux doigts constituent une fourche dont le positionnement par rapport à la cheville est particulièrement facile et reproductible. Cette autre paire de doigt constitue donc également une pince.

Ces réalisations sont donc basées sur l'utilisation de deux doigts de la main, soit du pouce et de l'index, soit du pouce et du majeur, soit du majeur et de l'index, pour réaliser la structure en pince nécessitée par l'invention pour venir placer les deux électrodes basses sur les deux points de la cheville sur la malléole interne Ci et sur la malléole externe Ce ainsi que montré schématiquement sur la figure 3B. Enfin, les réalisations présentées sur ces figures utilisent, pour les électrodes hautes, un contact soit sur l'index, soit au niveau du majeur et un contact sur le dos de la main.

Ainsi, la mécanique fonctionnelle de l'appareil est très réduite puisque la main remplit la majeure partie des fonctions mécaniques. L'utilisation des modes de réalisation présentés sur la figure 3 à 7 est donc particulièrement simplifiée et est donc parfaitement adaptée au grand public.

Par ailleurs, les réalisations présentées sur ces figures sont avantageusement réalisées à partir de matériaux flexibles simples, tels que des matériaux textiles ou encore des matériaux de type néoprène, caoutchouc synthétique, etc. L'utilisation des matériaux cités ci-dessus permet aussi de personnaliser aisément les appareils selon l'invention par un choix de matières et/ou d'inscriptions sur l'appareil.

Aussi, sans nuire aux caractéristiques de l'appareil selon l'invention, la couverture des doigts de la main pourra être variable, l'essentiel étant qu'au moins deux des doigts de la main soient couverts et portent les électrodes nécessaires à l'invention.

La figure 4 montre un exemple où le pouce et le majeur sont couverts par un matériau textile extensible prolongé jusqu'au poignet.

L'ensemble du matériau textile portant les deux extensions 11a et 11b constitue le support 10 sur lequel est placé le module électronique placé dans un boîtier 13. Le maintien du support 10 est assuré par une sangle 12, portant avantageusement une bande auto-aggripante et assurant le maintien de la main par rapport au support 10 et le contact d'une deuxième électrode haute 20a, représentée en pointillés, au niveau du dos de la main. La première électrode haute 20b, dont l'emplacement est également montré en pointillés, est placée à l'intérieur de l'extension 11b, sur sa face interne, et est en contact avec le majeur de l'utilisateur.

Deux électrodes basses 21a et 21b sont par ailleurs placées sur la face externe du support extensible, respectivement en extrémité du pouce et en extrémité de l'index en face à face avec l'électrode 20b, représentée par les pointillés.

On comprend que les extensions sont ici placées de manière à ce que le majeur et le pouce soient utilisés pour faire la mesure. Néanmoins, d'autres réalisations mettant en oeuvre le pouce et l'index pourront être utilisées.

Par ailleurs, ces réalisations permettent d'utiliser éventuellement des produits déjà existants et destinés à être placés sur la main, typiquement un gant, et d'y intégrer l'électronique nécessaire à l'appareil selon l'invention, c'est-à-dire le module et les électrodes.

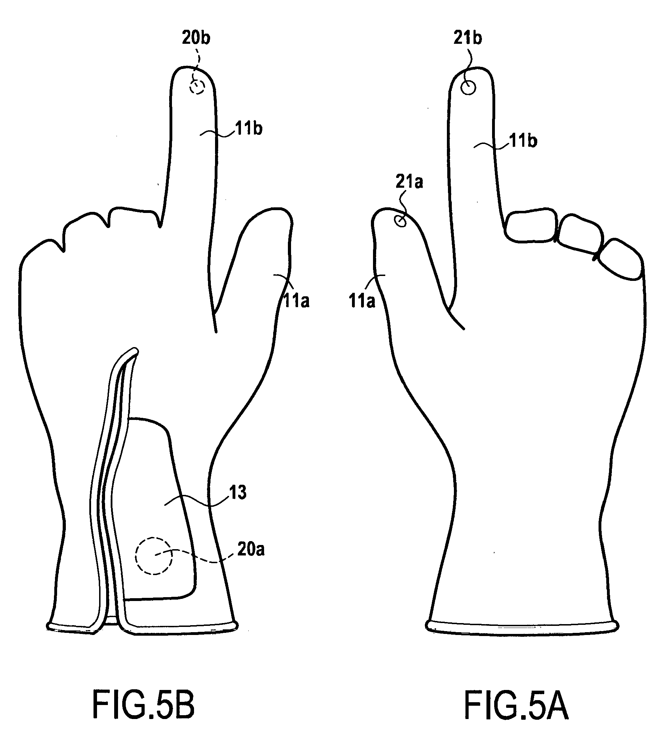

Les figures 5A et 5B montrent ainsi une réalisation utilisant le pouce et l'index à partir d'un gant vendu dans le commerce pour la pratique du golf. De tels gants présentent l'avantage d'être généralement vendu à l'unité et non par paire, c'est-à-dire soit le gant droit, soit le gant gauche.

On note ici que, dans le cas où des gants en matériau non élastique seraient utilisés, il sera indispensable de fabriquer plusieurs tailles pour permettre à toutes les tailles de main d'utiliser un appareil selon l'invention. Quand un matériau élastique est utilisé, il est possible de sous-dimensionner les extensions, de manière à ne prévoir qu'un petit nombre de dimensions pour satisfaire un large panel d'utilisateurs.

Dans tous les cas, la partie électronique intégrée reste la même et cela permet une fabrication peu coûteuse. Sur la figure 5B montrant le dos du gant, on voit que le gant présente une poche 13 faisant boîtier pour accueillir le module électronique ainsi directement intégré au sein du tissu du gant.

La figure 5A montre ainsi la paume d'un gant de golf, transformé en mitaine partielle recouvrant seulement l'index et le pouce et formant ainsi les deux extensions 11a et 11b sur lesquelles sont placées les électrodes 21a et 21b. La structure du gant constitue, elle-même, l'élément de maintien dans ce cas même si une sangle pourra être ajoutée pour assurer le contact avec la peau de l'utilisateur sur le dos de la main.

Eventuellement, il pourra être prévu que les doigts pleins soient réduits au maximum sur les deux dernières phalanges afin de réduire l'achalandage des tailles. Il pourra être aussi intéressant de rallonger la jupe au niveau du poignet. L'alvéole 13 pour l'emplacement du boîtier électronique sera avantageusement pratiquée entre deux couches de textile. Avantageusement, la matière textile sera composée de deux enveloppes pour permettre le passage des connexions des contacts.

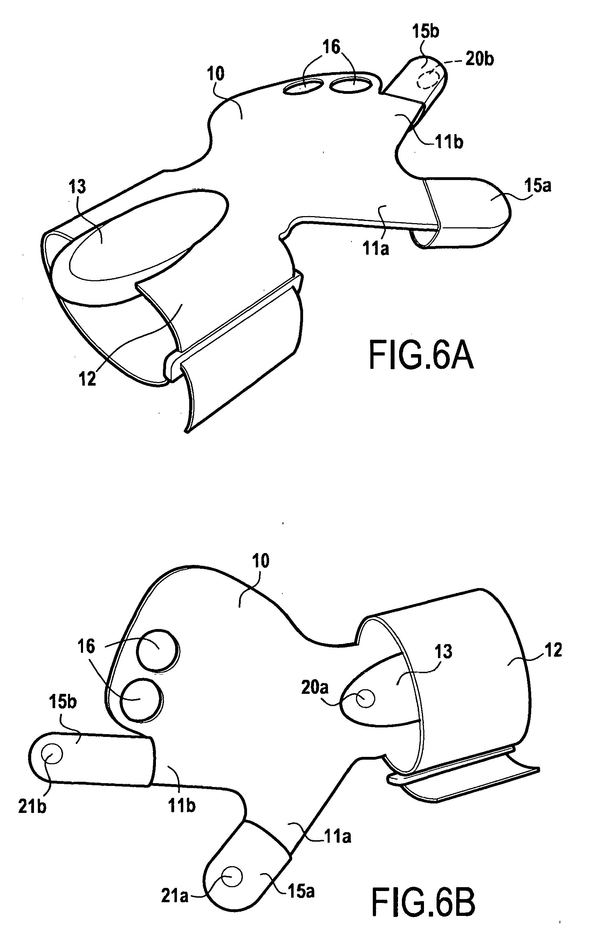

Les figures 6A et 6B montrent un mode de réalisation préférentiel selon l'invention. Dans ce mode de réalisation, un support monobloc 10 est simplement découpé dans une plaque de matériau souple. Le support 10 comprend ici une paume et deux extensions 11a et 11b, ainsi qu'un bracelet 12 directement découpé dans la plaque de matériau souple. Un boîtier 13 contenant le module électronique est fixé sur la plaque par des moyens connus de l'homme du métier.

Avantageusement, une des électrodes hautes 20a est directement portée par le boîtier 13, ainsi que cela est visible sur la figure 6B. L'élément de maintien 12 est avantageusement muni de bande auto-aggripante pour assurer la fermeture et le maintien du bracelet autour du poignet de l'utilisateur. La découpe de la plaque de matériau souple présente deux extensions 11a et 11b, à l'extrémité desquelles sont positionnés des capuchons 15a et 15b. Le capuchon 15a porte l'électrode basse 21a sur sa face externe, le capuchon 15b porte l'électrode basse 21b sur sa face externe et l'électrode haute 20B placée à l'intérieur du capuchon, afin de venir en contact avec l'index.

Dans cette réalisation, la plaque de matériau souple est destinée à être placée sur le dos de la main. Dans la réalisation préférentielle de la figure 6, la plaque comprend, en outre, deux orifices 16 de placement de deux autres doigts de la main, ici le majeur et l'annulaire. Ces orifices de placement permettent d'assurer un positionnement de la main tout à fait reproductible et précis.

Typiquement, la plaque de matériau souple présentera une texture caoutchouteuse ou néoprène et pourra être réalisée par découpage. Eventuellement, le matériau pourra être formé par coulage ou par formage.

Avantageusement, la plaque de matériau souple est composée de deux feuilles contrecollées qui offrent la possibilité d'une insertion préalable de toutes les connexions entre le module 13 et les électrodes hautes et basses.

Les capuchons 15a et 15b sont, avantageusement, des parties tubulaires fermées, elles-mêmes partiellement prises en sandwich entre les deux feuilles contrecollées composant le matériau souple. Les capuchons pourront être réalisés par moulage ou tissage synthétique.

Le module 13 est, avantageusement, placé dans une alvéole préformée sur le dessus de la main. Cette alvéole préformée est, avantageusement, aménagée au moment du contre-collage des deux feuilles constituant le matériau souple.

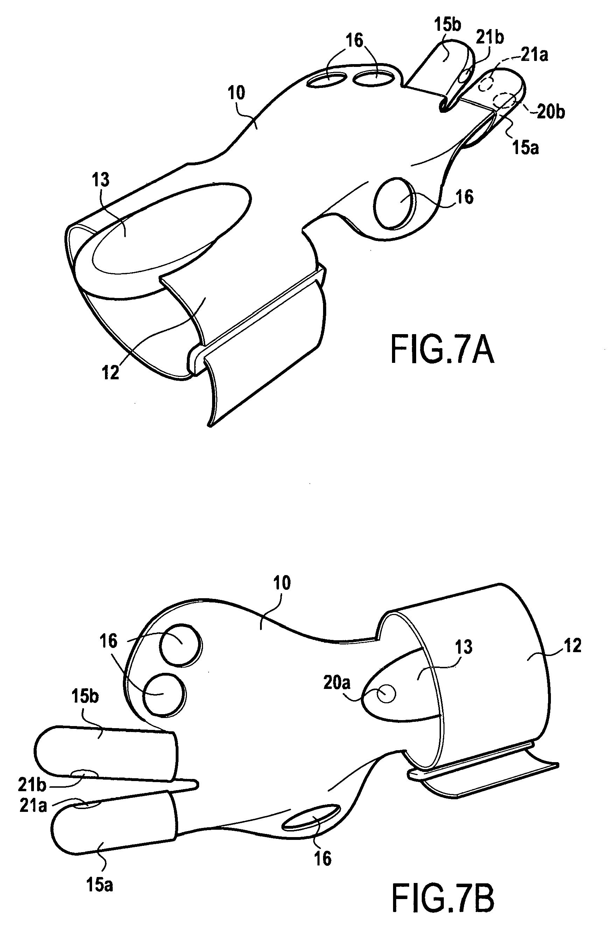

Les figures 7A et 7B montrent un sixième mode de réalisation selon le principe des réalisations préférentielles de l'invention.

Dans cette réalisation, ce sont le majeur et l'index qui sont utilisés pour placer les électrodes basses. Ces deux doigts forment en effet une fourche dont les deux piques sont de longueurs comparables. Dans ce cas, les électrodes basses 21a et 21b sont positionnées sur les tranches des deux doigts, index et majeur, se faisant face.

Le positionnement par rapport à la cheville est encore facilité car la courbure des doigts lors du positionnement de cette fourche sur la cheville n'est pas gênante car ne modifie pas la position des faces latérales des doigts contrairement à ce qui peut se passer avec le pouce. Les électrodes hautes 20a et 20b peuvent avantageusement conserver la même position au niveau du dos de la main et au niveau de l'index à l'intérieur du capuchon 15a.

Ce mode de réalisation est décrit sur le principe de la réalisation de la figure 6 avec un support découpé dans une plaque de matériau semi-rigide et des capuchons placés en extrémité des extensions. Cependant, on reconnaitra qu'un gant de golf recouvrant index et majeur ou qu'un dispositif souple du type de celui représenté sur la figure 4 et recouvrant index et majeur pourra aussi être mis en oeuvre selon le principe décrit sur la figure 7.

Lors de l'utilisation d'un appareil selon l'invention, l'utilisateur positionne le support, par exemple en enfilant un gant selon le mode de réalisation de la figure 5, puis met l'appareil sous tension et, à l'aide du pouce et de l'index, vient pincer l'arrière de la cheville. Avantageusement, la prise de mesure est automatique à partir de la détection des deux contacts sur les points de la cheville, c'est-à-dire sur les électrodes basses.

Avantageusement une diode électroluminescente ou un bip prolongé assurera le timing de l'opération pour signaler à l'utilisateur qu'il peut ôter sa main de sa cheville, une fois la prise de mesures réalisée.

Les mesures comprennent avantageusement quatre données : l'impédance à 5 kHz, l'impédance à 50 kHz, l'impédance à 200 kHz et l'angle de phrase à 50 kHz. Ces mesures ne seront représentatives que si les électrodes sont bien disposées, ce qui est précisément permis par l'invention. Avec l'objet de l'invention, les mesures sont obligatoirement prises dans des conditions identiques d'une fois sur l'autre, grâce à la facilité d'utilisation qui empêche que l'utilisateur se trompe dans l'utilisation du dispositif.

La mesure de bio-impédance est faite par une méthode quadripolaire en injectant un courant constant de faible valeur pour ne pas être perçu par le sujet. Le courant doit être ainsi de bonne qualité et la fréquence doit être précise. La mesure doit se faire en haute impédance et éliminer les basses fréquences d'origine secteur et biologique. La précision minimale de la mesure doit être de 2%. Selon les méthodes utilisées, la valeur mesurée peut varier de 10 à 2 000 Ω.

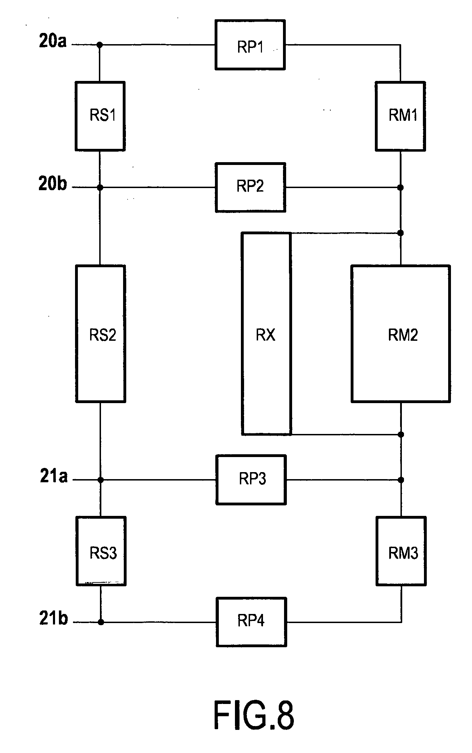

La figure 8 montre le principe de la bio-impédancemétrie quadripolaire. Les résistances qu'oppose le corps humain à la circulation du courant sont modélisées en trois colonnes correspondant, respectivement, à la résistance à la surface de la peau pour les résistances RS1, RS2, RS3, à la résistance de l'épaisseur de peau et du gras représentés par les résistances RP1, RP2, RP3, RP4, enfin, à la résistance des muscles modélisés par les résistances RM1, RM2, RM3.

Les résistances de contact au niveau de chaque électrode 20a, 20b, 21a, 21b, ne sont pas représentées sur cette figure. Les résistances sur toute la surface du corps sont notées RX. La bio-impédancemétrie permet de mesurer RM2 associé à RX par un courant constant établi entre les électrodes 20a et 21b. La mesure entre les électrodes 20b et 21a permet d'éliminer les résistances RP1 à RP4 et RS1 à RS3.

Si la peau n'est pas mouillée, on peut considérer que les valeurs de RS1 à RS3 sont négligeables car très grandes par rapport aux autres, ce qui permet de simplifier les représentations et, surtout, de rendre la mesure possible.

En fait, le corps est un élément conducteur hétérogène de résistivité variable et intégrant des parties réactives complexes et trois grandeurs physiques entrent dans la mesure de l'impédance : la résistance, la réactance et l'angle de phase. La résistance est bien connue. La réactance représente l'opposition au flux instantané de courant électrique causée par les éléments se comportant comme des condensateurs.

Appliquée à la membrane cellulaire, la réactance grandit avec la quantité de membranes. Une haute valeur de réactance indique l'intégrité de la membrane cellulaire. Théoriquement, la réactance est une mesure de la capacité volumique de la membrane cellulaire et une mesure indirecte du volume intracellulaire de la masse cellulaire du corps. Alors que le corps entier offre une résistance au passage de courant, il n'y a que la membrane qui offre une réactance. C'est le cas de la graisse dont la masse n'affecte pas la réactance globale.

L'angle de phase représente, quant à lui, une relation entre la résistance et la réactance : 0° correspond à un circuit résistant, 90° à un circuit capacitif, 45° correspond à autant de résistance que de capacité.

Pour un individu moyen, l'angle de phase varie de 3 à 10°. Une baisse de cet angle indique une baisse de la réactance, donc une mort cellulaire. Une hausse de cet angle indique une hausse de la réactance, donc une grande quantité de membranes cellulaires intactes.

Les mesures multifréquence permises par l'appareil selon l'invention permettent de connaître entre autre la quantité de protéines musculaires, l'eau intracellulaire, l'eau extracellulaire, la masse osseuse globale minérale et protéique et la masse grasse. La quantité totale d'eau est aussi accessible.

Avantageusement, l'appareil selon l'invention dispose de moyens de communication sans fil permettant de transmettre les données à tout support numérique. Ainsi les paramètres et résultats sont envoyés, avantageusement, par Bluetooth, Wifi ou port USB vers une destination de type PC, tablette, téléphone mobile ou autre. Les éléments de connexion à cette source externe sont placés au sein du boîtier 13 contenant le module électronique.

Par exemple, le module électronique est connecté/connectable par voie sans fil ou filaire au réseau Internet et, plus particulièrement, à un serveur qui permet le contrôle du poids et le suivi nutritionnel d'un sujet à son domicile via le réseau Internet. Un logiciel effectue alors des calculs et, avantageusement, donne l'interprétation des mesures en masse grasse, en masse musculaire, en métabolisme, etc.

Avec l'invention, l'utilisateur peut faire une mesure à domicile et se connecter aisément à une plateforme Internet pour partager les informations recueillies et en permettre l'analyse pour l'établissement d'une recommandation nutritionnelle et/ou un programme d'activité physique adapté à ses besoins et à ses capacités. La plateforme Internet dédiée permet alors le traitement des données et le suivi du sujet.

L'invention permet donc de produire des conseils nutritionnels et/ou d'activité physique très affinés par rapport au profil d'un sujet, en tout cas, bien plus que les sites actuellement connus qui proposent des conseils en fonction de l'indice de masse corporelle (IMC), qui est une donnée tout à fait insuffisante pour savoir si un sujet présente un problème de surpoids.

En effet, il n'est pas rare de trouver des sujets avec un IMC élevé, qui ne sont pas pour autant obèses. C'est notamment le cas des sportifs. Baser un programme nutritionnel sur la seule connaissance de l'IMC présente donc des dangers pour la santé de l'utilisateur qui pourrait suivre des recommandations qui ne le concernent pas. Seule la bio-impédancemétrie permet aujourd'hui d'évaluer et de suivre l'évolution du métabolisme de base, de la masse protéique des muscles et des os, de la masse graisseuse et de l'eau. L'invention permet d'ouvrir ces services de conseils personnalisés au grand public sans risque majeur encouru d'inadaptation des conseils puisque le profil de l'utilisateur est bien défini grâce à l'utilisation de mesures quadripolaires multifréquence de bio-impédancemétrie.

## Revendications

1. Appareil de prise de mesure de bio-impédancemétrie comprenant :
- un support (10) présentant deux extensions (11a, 11b), ce support étant destiné à accueillir une main d'un utilisateur et comprenant des moyens permettant le maintien du support dans une position prédéfinie par rapport à la main,
- deux électrodes (20a, 20b), dites hautes, supportées par le support et aptes à être mises en contact avec la peau en deux points distincts de la main lors du positionnement du support sur la main,
- deux électrodes (21a, 21b), dites basses, supportées chacune par une des extensions du support, et
- un module électronique supporté par le support et apte à commander les quatre électrodes pour réaliser une mesure de bio-impédancemétrie,
**caractérisé en ce que** :
- lesdites extensions forment une structure en pince adaptée pour pincer la cheville ou le pied de l'utilisateur par amenée de la main munie du support vers la cheville ou le pied, les deux électrodes basses (21a, 21b) étant alors, par l'action du pincement, mises en contact avec la peau en deux points distincts situés de part et d'autre de la cheville ou du pied, et
- ledit module comprend en outre des moyens d'émission des mesures de bio-impédancemétrie vers un dispositif de traitement des données et/ou d'affichage.

2. Appareil selon la revendication 1, **caractérisé en ce que** le support est tel qu'une des électrodes hautes (20a) est destinée à être positionnée au niveau du poignet et l'autre électrode haute (20b) est destinée à être positionnée au niveau d'un des doigts de la main, les deux électrodes étant séparées d'une distance supérieure ou égale à 11 centimètres sur le support (10).

3. Appareil selon l'une des revendications 1 et 2, **caractérisé en ce que** le module est installé dans un boîtier (13) apte à être positionné au niveau du poignet.

4. Appareil selon l'une des revendications 1 à 3, **caractérisé en ce que** les moyens permettant le maintien du support comprennent un élément de maintien (12), un bracelet ou une sangle, destiné à enserrer le poignet d'un utilisateur.

5. Appareil selon la revendication 4, **caractérisé en ce que** le bracelet ou la sangle (12), élastique ou non, supportent une des électrodes hautes (20a).

6. Appareil selon l'une des revendications 1 à 3, **caractérisé en ce que** les moyens permettant le maintien du support comprennent une poignée (14) destinée à être serrée dans la main d'un utilisateur.

7. Appareil selon l'une des revendications 1 à 3, **caractérisé en ce que** les moyens permettant le maintien du support sont constitués par la structure du support lui-même.

8. Appareil selon l'une des revendications précédentes, **caractérisé en ce qu'**une des deux extensions (11a, 11b) porte également une des électrodes hautes (20a, 20b), cette extension étant placée sur le support de telle manière que ledit support soit apte à recevoir un des doigts naturellement positionné au niveau de l'électrode lorsque la main est positionnée dans le support.

9. Appareil selon l'une des revendications précédentes, **caractérisé en ce que** les deux extensions (11a, 11b) constituent une pince flexible adaptée pour pincer la cheville ou le pied et mettre ainsi en contact les électrodes portées par les extensions en contact avec la peau de part et d'autre de la cheville ou du pied.

10. Appareil selon l'une des revendications 1 à 8, **caractérisé en ce que** les deux extensions (11a, 11b) sont dactylo-profilées pour recevoir deux des doigts de la main.

11. Appareil selon la revendication 10, **caractérisé en ce que** le support (10) a la forme d'un gant à au moins deux doigts, un des doigts du gant portant une électrode haute (20a, 20b) sur sa face interne et une électrode basse (21a) sur sa face externe, l'autre doigt portant une électrode basse (21b) sur sa face externe.

12. Appareil selon la revendication 10, **caractérisé en ce que** le support est réalisé à partir d'une plaque de matériau souple découpée selon une forme formant un bracelet de maintien (12) au niveau duquel est supporté le module et une première électrode haute (20a), une paume et deux extensions (11a, 11b) à l'extrémité desquelles sont placés des capuchons (15a, 15b) apte à recevoir les deux doigts de la main, un premier capuchon (15a) étant muni d'une électrode basse (21a) sur sa face externe, l'autre capuchon (15b) étant muni d'une électrode basse (21b) sur sa face externe et d'une électrode haute (20b) placée sur la face interne de ce capuchon.

13. Appareil selon la revendication 12, **caractérisé en ce que** la paume du support comprend au moins un orifice de placement (16) apte à recevoir un des autres doigts de la main, autre que ceux aptes à être introduits dans lesdits capuchons (15a, 15b).

14. Appareil selon l'une des revendications 10 à 13, **caractérisé en ce que** ladite paire de doigts est choisie parmi les paires suivantes : le pouce et l'index, le pouce et le majeur, l'index et le majeur.

15. Appareil selon l'une des revendications précédentes, **caractérisé en ce que** ledit module est apte à réaliser des mesures de bio-impédancemétrie multi-fréquentielles.

16. Appareil selon l'une des revendications précédentes, **caractérisé en ce que** le dispositif de traitement de données et/ou d'affichage est supporté par le support.

17. Appareil selon l'une des revendications précédentes, **caractérisé en ce que** les moyens d'émission comprennent des moyens de communication sans fil tels que l'appareil est relié par ces moyens de communication sans fil à un dispositif de traitement de données et/ou d'affichage.

18. Appareil selon la revendication 17, **caractérisé en ce que** le dispositif de traitement de données et/ou d'affichage est un serveur Internet.

## Patentansprüche

1. Gerät zur Bioimpedanz-Messung, umfassend:
- einen Träger (10), der zwei Erweiterungen (11 a, 11b) aufweist, wobei dieser Träger dazu bestimmt ist, eine Hand eines Benutzers aufzunehmen, und Mittel umfasst, die das Halten des Trägers in einer vordefinierten Position gegenüber der Hand ermöglichen,
- zwei sogenannte obere Elektroden (20a, 20b), die von dem Träger getragen und geeignet sind, beim Positionieren des Trägers an der Hand an zwei unterschiedlichen Stellen der Hand mit der Haut in Kontakt gebracht zu werden,
- zwei sogenannte untere Elektroden (21 a, 21 b), die jeweils von einer der Erweiterungen des Trägers getragen sind, und
- ein elektronisches Modul, das von dem Träger getragen und geeignet ist, die vier Elektroden zu steuern, um eine Bioimpedanz-Messung durchzuführen, **dadurch gekennzeichnet, dass**:
- die Erweiterungen eine Zangenstruktur bilden, die dazu ausgelegt ist, den Knöchel oder den Fuß des Benutzers dadurch zu umgreifen, dass die mit dem Träger versehene Hand zu dem Knöchel oder dem Fuß geführt wird, wobei die beiden unteren Elektroden (21 a, 21 b) dann durch die Wirkung des Umgreifens an zwei unterschiedlichen Stellen, die auf beiden Seiten des Knöchels oder des Fußes gelegen sind, mit der Haut in Kontakt gebracht werden, und
- das Modul ferner Mittel zum Senden der Messwerte der Bioimpedanz-Messung an eine Datenverarbeitungs- und/oder Anzeigevorrichtung umfasst.

2. Gerät nach Anspruch 1, **dadurch gekennzeichnet, dass** der Träger derart ist, dass eine der oberen Elektroden (20a) dazu bestimmt ist, im Bereich des Handgelenks positioniert zu werden, und die andere obere Elektrode (20b) dazu bestimmt ist, im Bereich von einem der Finger der Hand positioniert zu werden, wobei die beiden Elektroden um einen Abstand größer oder gleich 11 Zentimeter an dem Träger (10) getrennt sind.

3. Gerät nach einem der Ansprüche 1 und 2, **dadurch gekennzeichnet, dass** das Modul in einem Gehäuse (13) angebracht ist, welches geeignet ist, im Bereich des Handgelenks positioniert zu werden.

4. Gerät nach einem der Ansprüche 1 bis 3, **dadurch gekennzeichnet, dass** die Mittel, die das Halten des Trägers ermöglichen, ein Halteelement (12), ein Armband oder einen Gurt, umfassen, welches dazu bestimmt ist, das Handgelenk eines Benutzers zu umschließen.

5. Gerät nach Anspruch 4, **dadurch gekennzeichnet, dass** das Armband oder der Gurt (12), elastisch oder nicht, eine der oberen Elektroden (20a) trägt.

6. Gerät nach einem der Ansprüche 1 bis 3, **dadurch gekennzeichnet, dass** die Mittel, die das Halten des Trägers ermöglichen, einen Griff (14) umfassen, der dazu bestimmt ist, in der Hand eines Benutzers festgehalten zu werden.

7. Gerät nach einem der Ansprüche 1 bis 3, **dadurch gekennzeichnet, dass** die Mittel, die das Halten des Trägers ermöglichen, durch die Struktur des Trägers selbst gebildet sind.

8. Gerät nach einem der vorhergehenden Ansprüche, **dadurch gekennzeichnet, dass** eine der beiden Erweiterungen (11a, 11 b) auch eine der oberen Elektroden (20a, 20b) trägt, wobei diese Erweiterung an dem Träger derart angeordnet ist, dass der Träger geeignet ist, einen der Finger aufzunehmen, der auf natürliche Weise im Bereich der Elektrode platziert ist, wenn die Hand in dem Träger positioniert ist.

9. Gerät nach einem der vorhergehenden Ansprüche, **dadurch gekennzeichnet, dass** die beiden Erweiterungen (11a, 11b) eine flexible Zange bilden, die dazu ausgelegt ist, den Knöchel oder den Fuß zu umgreifen und somit die von den Erweiterungen getragenen Elektroden auf beiden Seiten des Knöchels oder des Fußes mit der Haut in Kontakt zu bringen.

10. Gerät nach einem der Ansprüche 1 bis 8, **dadurch gekennzeichnet, dass** die beiden Erweiterungen (11a, 11b) fingerprofiliert sind, um zwei der Finger der Hand aufzunehmen.

11. Gerät nach Anspruch 10, **dadurch gekennzeichnet, dass** der Träger (10) die Form eines Handschuhs mit wenigstens zwei Fingern hat, wobei einer der Finger des Handschuhs eine obere Elektrode (20a, 20b) auf seiner Innenseite und eine untere Elektrode (21 a) auf seiner Außenseite trägt, wobei der andere Finger eine untere Elektrode (21 b) auf seiner Außenseite trägt.

12. Gerät nach Anspruch 10, **dadurch gekennzeichnet, dass** der Träger aus einer Platte aus flexiblem Material gefertigt ist, die entsprechend einer Form zugeschnitten ist, welche ein Haltearmband (12), in dessen Bereich das Modul und eine erste obere Elektrode (20a) getragen sind, einen Handteller sowie zwei Erweiterungen (11a, 11b) bildet, an deren Ende Kappen (15a, 15b) angeordnet sind, die geeignet sind, die beiden Finger der Hand aufzunehmen, wobei eine erste Kappe (15a) mit einer unteren Elektrode (21 a) auf ihrer Außenseite versehen ist, wobei die andere Kappe (15b) mit einer unteren Elektrode (21 b) auf ihrer Außenseite und mit einer oberen Elektrode (20b), die auf der Innenseite dieser Kappe angeordnet ist, versehen ist.

13. Gerät nach Anspruch 12, **dadurch gekennzeichnet, dass** der Handteller des Trägers wenigstens eine Platzierungsöffnung (16) umfasst, die geeignet ist, einen der anderen Finger der Hand aufzunehmen, einen anderen als diejenigen, die geeignet sind, in die Kappen (15a, 15b) eingeführt zu werden.

14. Gerät nach einem der Ansprüche 10 bis 13, **dadurch gekennzeichnet, dass** das Fingerpaar aus den folgenden Paaren ausgewählt ist: dem Daumen und dem Zeigefinger, dem Daumen und dem Mittelfinger, dem Zeigefinger und dem Mittelfinger.

15. Gerät nach einem der vorhergehenden Ansprüche, **dadurch gekennzeichnet, dass** das Modul geeignet ist, Bioimpedanz-Mehrfrequenzmessungen durchzuführen.

16. Gerät nach einem der vorhergehenden Ansprüche, **dadurch gekennzeichnet, dass** die Datenverarbeitungs- und/oder Anzeigevorrichtung von dem Träger getragen ist.

17. Gerät nach einem der vorhergehenden Ansprüche, **dadurch gekennzeichnet, dass** die Sendemittel drahtlose Kommunikationsmittel umfassen, die derart sind, dass das Gerät durch diese drahtlosen Kommunikationsmittel mit einer Datenverarbeitungs- und/oder Anzeigevorrichtung verbunden ist.

18. Gerät nach Anspruch 17, **dadurch gekennzeichnet, dass** die Datenverarbeitungs- und/oder Anzeigevorrichtung ein Internetserver ist.

## Claims

1. An appliance for taking a bioimpedance measurement, the appliance comprising:
· a support (10) presenting two extensions (11a, 11b), the support being designed to receive a hand of a user and including means enabling the support to be held in a predefined position relative to the hand;
· two high electrodes (20a, 20b) borne by the support and suitable for being put into contact with the skin at two distinct points of the hand while the support is being positioned on the hand;
· two low electrodes (21a, 21b), each borne by one of the extensions of the support; and
· an electronic module borne by the support and suitable for controlling the four electrodes in order to perform a bioimpedance measurement;
**characterized in that**:
· said extensions form a pincer structure suitable for pinching the ankle or the foot of the user by bringing the hand fitted with the support to the ankle or the foot, the two low electrodes (21a, 21b) then being put into contact, by the pinching action, with the skin at two distinct points situated on either side of the ankle or the foot; and
· said module also comprises means for transmitting bioimpedance measurements to a data processor and/or display device.

2. An appliance according to claim 1, **characterized in that** the support is such that one of the high electrodes (20a) is for positioning on the wrist and the other high electrode (20b) is for positioning on one of the digits of the hand, the two electrodes being spaced apart on the support (10) by a distance that is greater than or equal to 11 centimeters.

3. An appliance according to claim 1 or claim 2, **characterized in that** the module is installed in a housing (13) suitable for being positioned at the level of the wrist.

4. An appliance according to any one of claims 1 to 3, **characterized in that** the means for holding the support comprise a holder element (12), a band or a strap, intended to encircle the wrist of a user.

5. An appliance according to claim 4, **characterized in that** the band or strap (12), elastic or non-elastic, bears one of the high electrodes (20a).

6. An appliance according to any one of claims 1 to 3, **characterized in that** the means for holding the support comprise a handle (14) that is intended to be held in the hand of a user.

7. An appliance according to any one of claims 1 to 3, **characterized in that** the means for holding the support are constituted by the structure of the support itself.

8. An appliance according to any preceding claim, **characterized in that** one of the two extensions (11a, 11b) also carries one of the high electrodes (20a, 20b), this extension being placed on the support in such a manner that said support is suitable for receiving one of the fingers that is naturally positioned level with the electrode when the hand is positioned in the support.

9. An appliance according to any preceding claim, **characterized in that** the two extensions (11a, 11b) constitute a flexible pincer adapted to pinch the ankle or the foot, thereby putting the electrodes carried by the extensions into contact with the skin on both sides of the ankle or the foot.

10. An appliance according to any one of claims 1 to 8, **characterized in that** the two extensions (11a, 11b) have the shape of fingers in order to receive two fingers of the hand.

11. An appliance according to claim 10, **characterized in that** the support (10) is in the form of a glove with at least two fingers, one of the fingers of the glove carrying a high electrode (20a, 20b) on its inside face and a low electrode (21a) on its outside face, the other finger carrying a low electrode (21b) on its outside face.

12. An appliance according to claim 10, **characterized in that** the support is made from a sheet of flexible material cut to form a holder band (12) at the level of which the module and a first high electrode (20a) are borne, a palm and two extensions (11a, 11b) having tips (15a, 15b) placed at their ends, the tips being suitable for receiving two fingers of the hand, a first tip (15a) being provided with a low electrode (21a) on its outside face, the other tip (15b) being provided with a low electrode (21b) on its outside face and a high electrode (20b) placed on the inside face of the tip.

13. An appliance according to claim 12, **characterized in that** the palm of the support comprises at least one placement orifice (16) suitable for receiving one of the other fingers of the hand, other than those suitable for being inserted in said tips (15a, 15b).

14. An appliance according to any one of claims 10 to 13, **characterized in that** said pair of fingers is selected from the following pairs: the thumb and the index finger; the thumb and the middle finger; the index finger and the middle finger.

15. An appliance according to any preceding claim, **characterized in that** said module is suitable for performing multifrequency bioimpedance measurements.

16. An appliance according to any preceding claim, **characterized in that** the data processor and/or display device is borne by the support.

17. An appliance according to any preceding claim, **characterized in that** the means for transmitting means comprise wireless communication means such that the appliance is connected by said wireless communication means with a data processor and/or display device.

18. An appliance according to claim 17, **characterized in that** the data processor and/or display device is an Internet server.
